# EUROPÄISCHE PATENTSCHRIFT

(11) **EP 2 292 148 B1**
(45) Veröffentlichungstag und Bekanntmachung des Hinweises auf die Patenterteilung: **24.08.2011**
(21) Anmeldenummer: 09011358.0
(22) Anmeldetag: 04.09.2009
(51) Int. Cl.: A61B 17/04, A61F 2/08

(54) **Knochenanker**
Bone anchor
Système d'ancrage osseux

(43) Veröffentlichungstag der Anmeldung: 09.03.2011
(73) Patentinhaber: IST Innovative Shoulder Technology AG, 8808 Pfäffikon (CH)
(72) Erfinder: Kropf, Philipp, 6330 Cham (CH); Leuzinger, Jan, 8808 Pfäffikon (CH)
(74) Vertreter: Schalch, Rainer

(56) Entgegenhaltungen:
- EP-A2- 1 800 608
- WO-A1-94/28811
- WO-A1-97/10767
- DE-A1- 2 747 312
- US-A- 5 626 613
- US-A- 5 628 766

## Beschreibung

Die Erfindung betrifft einen Knochenanker gemäss Oberbegriff des Anspruchs 1.

Bei verschiedenen human- und veterinärchirurgischen Verfahren müssen Weichteile wie beispielsweise Sehnen und Bänder, an einem Knochen fixiert werden. Das Fixieren kann beispielsweise dann erforderlich sein, wenn auf Grund eines Unfalls oder eines degenerativen Prozesses eine Sehne von einem Knochen abgelöst ist und die Sehne nun wieder fixiert werden muss. Zur Fixierung der Weichteile werden in der Chirurgie üblicherweise verschiedene Vorrichtungen eingesetzt, die typischerweise als Knochenanker bezeichnet werden. Ein solcher Knochenanker wird in einen freigelegten Knochen eingebracht, wobei mit einem am Knochenanker fixierten bzw. von dem Knochenanker geführten Fadenmaterial - üblicherweise ein oder mehrere Fäden - beispielsweise die abgelöste Sehne am Knochenanker und damit am Knochen befestigt wird.

Ein bekannter Knochenanker ist der so genannte Statak-Knochenanker des Unternehmens Zimmer. Der Statak-Knochenanker umfasst einen zylinderförmigen Schraubenkörper mit einem selbstschneidenden Aussengewinde, einer distalen kegelförmigen Ankerspitze und einem proximalen Kopfteil. Das Aussengewinde erstreckt sich im Wesentlichen zwischen der Ankerspitze und dem Kopfteil. Der Kopfteil weist eine Öse zur Aufnahme des Fadenmaterials auf, die sich senkrecht zur Längsachse des Schraubenkörpers erstreckt. Der Kopfteil hat ferner eine mehrkantige äussere Oberfläche zum Ansetzen eines Werkzeugs, weshalb der Kopfteil auch kein Aussengewinde hat. Bei dem Statak-Knochenanker handelt es sich um einen Schraubanker, der in den Knochen geschraubt wird.

Neben solchen Schraubankern werden auch Einschlaganker verwendet, die mit einem speziellen Setzinstrument/Werkzeug in den Knochen eingeschlagen werden. Es können vom zylinderförmigen Körper des Knochenankers abstehende Flügel oder Widerhaken vorgesehen sein, die sich unter dem kortikalen Knochen verkeilen und so den Knochenanker sicher im Knochen halten sollen. Am proximalen Ende hat der als Einschlaganker ausgeführte Knochenanker üblicherweise quer zu seiner Längsachse eine Durchführung zur Aufnahme eines Haltefadens. Ein Beispiel für einen solchen Knochenanker ist der so genannte G2-Anker von De-Puy Mitek.

Ein weiterer bekannter Knochenanker ist der so genannte Tag-Rod-Anker von Smith Nephew. Bei diesem Anker werden die Fäden durch eine Öse geführt, welche die Spitze des Ankers bildet. Nach einem Vorbohren wird dieser Tag-Rod-Anker in den Knochen eingeschlagen. Der Tag-Rod-Anker weist an seinem Körper ringförmige Erhebungen auf, die dazu führen, dass der Tag-Rod-Anker nach dem Einschlagen im weichen spongiösen Knochen Widerhalt findet.

Ein weiterer bekannter Knochenanker ist der so genannte Pushlock-Anker von Arthrex. Bei diesem Anker werden vorgelegte Fäden, die bereits durch die Sehne führen, durch eine an die distale Spitze des Ankers anschliessende Öse geführt. Der Anker wird dann durch den kortikalen Knochen eingeschlagen. Anschliessend werden die Fäden über einen über die Öse geschobenen Dübel aus Polyethylen fixiert.

US 5 628 766 bildet die Basis für den Oberbegriff des Hauptanspruchs.

Mediziner, insbesondere Chirurgen, erwarten von Knochenankern, dass sie zuverlässig implantierbar und praktisch handhabbar sind. So sollen zum Beispiel die mit dem Knochenanker verwendeten Fäden leicht einzufädeln sein, nicht ungewollt abreissen und sich nicht verheddern. Ausserdem soll der Knochenanker einen sicheren Halt im Knochen gewährleisten. Ferner soll der Knochenanker bei schlechter Lage im Knochen auch wieder entfernt werden können. Bei den handelsüblichen Einschlagankern besteht der Nachteil, dass diese nicht auf einfachem Wege mit einem einfach ausgestalteten Werkzeug wieder aus dem Knochen entfernt werden können. So kann beispielsweise der G2-Anker wegen seiner Widerhaken erst nach Entfernen des kortikalen Knochens entfernt werden. Bei den anderen bekannten Einschlagankern verhält es sich ähnlich.

Es ist Aufgabe der Erfindung, einen Knochenanker zur Befestigung von Weichteilen an einem Knochen, der Kortikalis und spongiöse Anteile aufweist, bereitzustellen, der als Einschlaganker eingesetzt werden kann und der sich gegenüber den oben genannten bekannten Einschlagankern durch eine verbesserte interoperative Handhabbarkeit auszeichnet. Es ist ferner Aufgabe der Erfindung, einen Knochenanker zur Befestigung von Weichteilen an einem Knochen, der Kortikalis und spongiöse Anteile aufweist, bereitzustellen, der als Einschlaganker eingesetzt werden kann und der einen sicheren Halt im Knochen gewährleistet, während er sich bei Bedarf relativ leicht wieder interoperativ aus dem Knochen entfernen lässt.

Die Aufgabe wird durch einen Knochenanker mit den Merkmalen des Anspruchs 1 gelöst.

Der erfindungsgemässe Knochenanker umfasst einen Körper mit einem proximalen Ende und einem distalen Ende. Ausgehend vom proximalen Ende weist der Körper einen ersten Abschnitt und einen zweiten, sich an den ersten Abschnitt anschliessenden Abschnitt auf. Bei dem distalen Ende handelt sich um das Ende des Körpers, das beim in einen Knochen implantierten Knochenanker tiefer im Knochen sitzt. Bei dem proximalen Ende handelt es sich um das dem distalen Ende gegenüberliegende Ende, das im implantierten Zustand des Knochenankers näher zur Knochenoberfläche liegt. Der zweite Abschnitt weist eine Einrichtung zur Aufnahmen eines Fadens (auch Haltefaden genannt) auf, bei der es sich vorzugsweise um eine Öse handelt. Auf der Mantelfläche des ersten Abschnitts des Körpers ist eine helixförmige bzw. wendelförmige Erhebung aufgebracht bzw. vorgesehen, die sich um die Mantelfläche des ersten Abschnitts windet. Der zweite Abschnitt weist keine helixförmige oder sonstige Erhebung auf, sodass die Mantelfläche des zweiten Abschnitts bis auf die Öse eben ist. Die helixförmige Erhebung des ersten Abschnitts des Körpers hat ein abgerundetes, gekrümmtes Profil, das bevorzugterweise von der Längsachse des Knochenankers wegweist.

Bei dem erfindungsgemässen Knochenanker handelt es sich insbesondere um einen Einschlaganker. Zum Implantieren des erfindungsgemässen Knochenankers in einem Knochen wird der Knochen vorzugsweise derart vorgebohrt, dass in dem Knochen eine Bohrung entsteht, deren Durchmesser dem Aussendurchmesser des Knochenankers ohne helixförmige Erhebung entspricht. Die zu fixierenden Fäden, die an die zu befestigenden Weichteile angebracht worden sind, werden in die Einrichtung zur Aufnahme eines Fadens, die insbesondere als Öse ausgestaltet ist, eingebracht. Anschliessend wird der erfindungsgemässe Knochenanker mittels eines geeigneten Werkzeugs in die vorgebohrte Bohrung eingeschlagen. Wegen des abgerundeten Profils der helixförmigen Erhebung lässt sich der erfindungsgemässe Knochenanker einfach und schnell in einen Knochen einschlagen, wobei die helixförmige Erhebung den Knochenanker nach dem Einschlagen sicher im Knochen hält und gegen ein ungewolltes Herausrutschen im Knochen fixiert. Sollte sich im Nachhinein herausstellen, dass der erfindungsgemässe Knochenanker nicht optimal im Knochen sitzt, so kann er wegen der helixförmigen Erhebung einfach mittels eines geeigneten Werkzeugs durch Herausdrehen wieder aus dem Knochen entfernt werden und danach wieder neu implantiert werden. Auf diese Weise kann vermieden werden, dass der Knochenanker bei insuffizienter Lage verloren geht.

Weitere vorteilhafte Ausgestaltungen der Erfindung ergeben sich aus den abhängigen Ansprüchen und den anhand der Zeichnungen nachfolgend dargestellten Ausführungsbeispielen. Es zeigen:
Fig. 1 eine Seitenansicht des erfindungsgemässen Knochenankers,
Fig. 2 eine Draufsicht auf den in Fig. 1 dargestellten Knochenanker,
Fig. 3 eine gegenüber der in Figur 1 gezeigten Seitenansicht um 90 Grad um die Längsachse gedrehte Seitenansicht des in Fig. 1 dargestellten Knochenankers und
Fig. 4 eine perspektivische Darstellung des in den Fig. 1 bis 3 dargestellten Knochenankers.

In den Figuren bezeichnen gleiche Bezugszeichen gleiche oder gleich wirkende Komponenten. Die Dimensions- und Massangaben in den Figuren sind rein beispielhafter Natur und ihre Einheit "mm" ist der Einfachheit halber in den Figuren weggelassen.

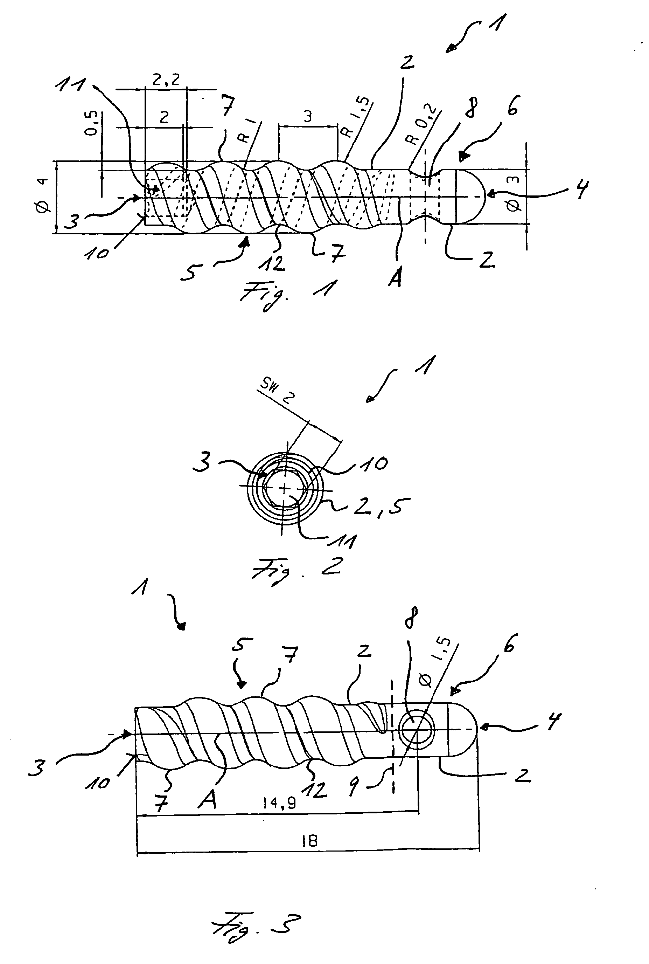

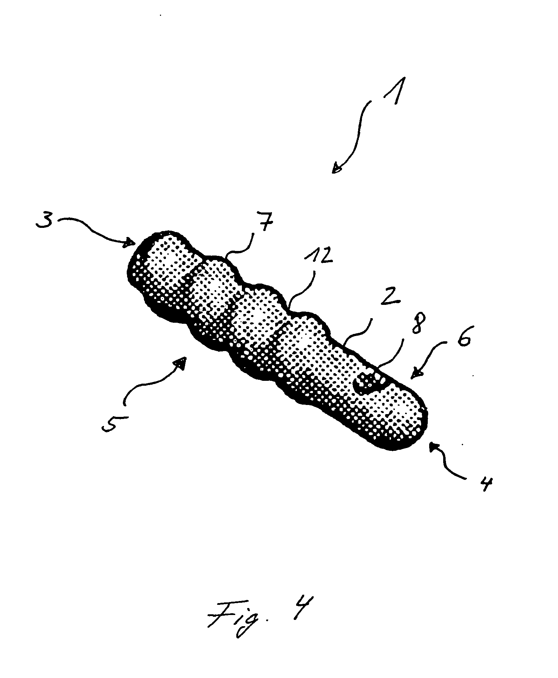

Die Figuren 1 bis 4 zeigen einen erfindungsgemässen Knochenanker 1, der zur Befestigung von Weichteilen an einem menschlichen oder tierischen Knochen dient und der einen Körper 2 umfasst, der sich symmetrisch um seine Längsachse A von einem proximalen Ende 3 zu einem distalen Ende 4 erstreckt. Der Körper 2 ist vorzugsweise zylinderförmig ausgestaltet. Der Körper 2 kann jedoch beispielsweise auch die Form eines Kegelstumpfes aufweisen, der sich vom proximalen Ende 3 ausgehend in Richtung auf das distale Ende 4 verjüngt.

Der Körper 2 des Knochenankers 1 weist einen ersten, vom proximalen Ende 3 ausgehenden Abschnitt 5 und einen zweiten, sich an den ersten Abschnitt 5 anschliessenden und sich bis zum distalen Ende 4 erstreckenden Abschnitt 6 auf. Der erste Abschnitt 5 hat auf seiner nicht näher bezeichneten Mantelfläche eine helixförmige Erhebung 7, die die Mantelfläche des ersten Abschnitts 5 umläuft. Das Profil der helixförmigen Erhebung 7 ist abgerundet bzw. die helixförmige Erhebung 7 weist von der Mantelfläche wegweisend einen abgerundeten Querschnitt auf. Die helixförmige Erhebung 7 ist somit nicht selbstschneidend. Durch die helixförmige Erhebung 7 erhält ein implantierter Knochenanker 1 sicheren Halt im Knochen.

Der zweite Abschnitt 6 weist eine Öse 8 auf, durch die ein Faden geführt werden kann, der an einem Weichteil befestigt ist bzw. befestigt werden soll. Die Öse 8 erstreckt sich senkrecht zur Längsrichtung durch den zweiten Abschnitt 6. Die nicht näher bezeichnete Mantelfläche des zweiten Abschnitts 6 ist bis auf die Öse 8 eben und weist keine Erhebung auf.

Der erste Abschnitt 5 hat insbesondere eine Länge zwischen 6 und 16 mm, wobei der zweite Abschnitt 6 insbesondere eine Länge von 2 bis 5 mm hat. Der Innendurchmesser der Öse 8 beträgt vorzugsweise 1.5 mm. Das von der Mantelfläche des ersten Abschnitts 5 wegweisende Profil der helixförmigen Erhebung 7 bzw. ihr Querschnitt weist vorzugsweise einen Radius zwischen 1 und 1.5 mm auf. Die Übergänge 12 zwischen den einzelnen Windungen der helixförmigen Erhebung 7 sind in Seitenansicht gesehen (vgl. Figuren 1 und 3) vorzugsweise ebenfalls gekrümmt, wobei die Krümmung nach innen zur Mantelfläche bzw. Längsachse A des ersten Abschnitts 5 hin ist. Die Übergänge 12 weisen vorzugsweise einen Radius von 1 mm auf, wobei der Abstand zwischen den Maxima zweier benachbarter Windungen der helixförmigen Erhebung 7 vorzugsweise 3 mm beträgt.

Der Körper 2 kann einstückig ausgeführt sein, sodass der erste Abschnitt 5 und der zweite Abschnitt 6 drehfest miteinander verbunden sind. Bevorzugt wird der Körper 2 aus zwei separaten Abschnitten 5 und 6 gebildet, wobei der erste Abschnitt 5 und der zweite Abschnitt 6 derart miteinander verbunden sind, dass sie gegeneinander beweglich, insbesondere frei drehbar, sind, sodass der zweite Abschnitt 6 nicht mitdreht, wenn der erste Abschnitt 5 um die Längsachse A gedreht wird. Die zweiteilige Ausgestaltung des Körpers 2 ist in Figur 3 durch die gestrichelte Linie 9 angedeutet. Soll ein implantierter Knochenanker 1 z.B. wegen insuffizienter Lage aus einem Knochen herausgedreht werden, so bietet die zweiteilige Ausgestaltung den Vorteil, dass sich zwar der erste Abschnitt 5 dreht, dass sich aber der zweite Abschnitt 6 nicht mit dem ersten Abschnitt 5 mitdreht, sodass ein Reissen, ein Verheddern oder eine sonstige Beeinträchtigung der in die Öse 8 des zweiten Abschnitts 6 eingebrachten Fäden vermieden werden kann.

Am proximalen Ende 3 des Knochenankers 1 weist die Aussenfläche 10 des ersten Abschnitts 5, die senkrecht zur nicht näher bezeichneten Mantelfläche des ersten Abschnitts 5 verläuft, eine Vertiefung 11 zum Ansetzen eines Werkzeugs (auch Führungs- oder Setzgerät bzw. -instrument genannt) auf, die sich vorzugsweise entlang der Längsachse A erstreckt und symmetrisch zu dieser ist. In die Vertiefung 11 kann ein entsprechendes Werkzeug eingebracht werden, um den Knochenanker 1 zu führen und/oder zu drehen. Die Vertiefung 11 hat insbesondere eine Form, die ein Herausdrehen eines implantierten Knochenankers 1 aus einem Knochen durch Rotation des Knochenankers 1 um seine Längsachse A erlaubt, wobei ein als Schraubendreher ausgeführtes Werkzeug als Ausdreher verwendet werden kann. Die Vertiefung 11 hat daher beispielsweise die Form einen Innenvierkants, eines Innensechskants oder eines so genannten Tork-Inlays, sodass ein entsprechendes Werkzeug in Form eines Innenvierkantschlüssels, eines Innensechskantschlüssels oder eines Tork-Stabs verwendet werden kann.

Der Körper 2 besteht vorzugsweise aus einem biokompatiblen Material wie beispielsweise Edelstahl, Titan, einer Titanlegierung, einem oder mehreren resorbierbaren Werkstoffen oder einem oder mehreren nichtresorbierbaren Werkstoffen (wie z.B. Peek, Polyethylen, Carbon). Selbstverständlich kann der Körper 2 auch aus einer Kombination der im letzten Satz aufgezählten Materialen miteinander oder mit anderen Materialien bestehen.

## Patentansprüche

1. Knochenanker zur Befestigung von Weichteilen an einem Knochen, der einen Körper (2) mit einem proximalen Ende (3) und einem distalen Ende (4) aufweist, wobei der Körper (2) einen ersten, vom proximalen Ende (3) ausgehenden Abschnitt (5) und einen zweiten, sich an den ersten Abschnitt (5) anschliessenden Abschnitt (6) aufweist, wobei der zweite Abschnitt (6) eine Einrichtung (8) zur Aufnahme eines Fadens aufweist, **dadurch gekennzeichnet, dass** auf der Mantelfläche des ersten Abschnitts (5) eine helixförmige Erhebung (7) angebracht ist, die sich um die Mantelfläche des ersten Abschnitts (5) windet, wobei die helixförmige Erhebung (7) ein abgerundetes Profil hat, und dass der zweite Abschnitt (6) keine helixförmige oder sonstige Erhebung aufweist.

2. Knochenanker nach Anspruch 1, **dadurch gekennzeichnet, dass** das abgerundete Profil einen Radius zwischen 1 und 1.5 mm hat.

3. Knochenanker nach Anspruch 1 oder 2, **dadurch gekennzeichnet, dass** der erste Abschnitt (5) eine Länge von 6 bis 16 mm hat.

4. Knochenanker nach einem der vorhergehenden Ansprüche, **dadurch gekennzeichnet, dass** der zweite Abschnitt (6) eine Länge von 2 bis 5 mm hat.

5. Knochenanker nach einem der vorhergehenden Ansprüche, **dadurch gekennzeichnet, dass** der Körper (2) zylinderförmig oder kegelstumpfförmig und sich vom proximalen Ende (3) in Richtung auf das distale Ende (6) verjüngend ist.

6. Knochenanker nach einem der vorhergehenden Ansprüche, **dadurch gekennzeichnet, dass** die Einrichtung (8) zur Aufnahme eines Fadens als Öse ausgestaltet ist.

7. Knochenanker nach einem der vorhergehenden Ansprüche, **dadurch gekennzeichnet, dass** der Körper (2) einstückig ausgeführt ist.

8. Knochenanker nach einem der vorhergehenden Ansprüche, **dadurch gekennzeichnet, dass** der erste Abschnitt (5) und der zweite Abschnitt (6) voneinander separate, miteinander verbundene Elemente sind, die gegeneinander beweglich, insbesondere drehbar, sind.

9. Knochenanker nach einem der vorhergehenden Ansprüche, **dadurch gekennzeichnet, dass** an dem proximalen Ende (3) eine Vertiefung (11) zum Ansetzen eines Werkzeugs vorgesehen ist.

10. Knochenanker nach Anspruch 9, **dadurch gekennzeichnet, dass** die Vertiefung (11) eine Form hat, die ein Herausdrehen eines in einen Knochen implantierten Knochenankers (1) aus dem Knochen mit Hilfe eines entsprechenden, in die Vertiefung (11) eingebrachten Werkzeugs erlaubt.

11. Knochenanker nach Anspruch 10, **dadurch gekennzeichnet, dass** die Vertiefung (11) die Form eines Innenvierkants oder eines Innensechskants hat.

## Claims

1. Bone anchor for attachment of soft parts to a bone, which has a body (2) with a proximal end (3) and a distal end (4), wherein the body (2) has a first section (5) starting from the proximal end (3) and a second section (6) that is attached to the first section (5), wherein the second section (6) comprises a device (8) for receiving a thread, **characterized in that** on the lateral surface area of the first section (5) a helix-shaped projection (7) is arranged that winds around the lateral surface area of the first section (5), wherein the helix-shaped projection (7) has a rounded profile, and that the second section (6) has no helix-shaped or other projection.

2. Bone anchor according to claim 1, **characterized in that** the rounded profile has a radius between 1 and 1.5 mm.

3. Bone anchor according to claim 1 or 2, **characterized in that** the first section (5) has a length from 6 to 16 mm.

4. Bone anchor according to one of the preceding claims, **characterized in that** the second section (6) has a length from 2 to 5 mm.

5. Bone anchor according to one of the preceding claims, **characterized in that** the body (2) is cylindrical or frustoconical and is tapered from the proximal end (3) in the direction to the distal end (6).

6. Bone anchor according to one of the preceding claims, **characterized in that** the device (8) for receiving the thread is designed as eyelet.

7. Bone anchor according to one of the preceding claims, **characterized in that** the body (2) is formed integrally.

8. Bone anchor according to one of the preceding claims, **characterized in that** the first section (5) and the second section (6) are elements that are separate from each other and connected with each other, which can be moved, in particular rotated, relative to each other.

9. Bone anchor according to one of the preceding claims, **characterized in that** a recess (11) is provided at the proximal end (3) for applying a tool.

10. Bone anchor according to claim 9, **characterized in that** the recess (11) has a form that allows for unscrewing of a bone anchor (1) implanted into a bone from the bone by means of a corresponding tool that has been positioned in the recess (11).

11. Bone anchor according to claim 10, **characterized in that** the recess (11) has the form of an inner square or an inner hexagon.

## Revendications

1. Système d'ancrage osseux pour la fixation de parties molles à un os, lequel système comporte un corps (2) avec une extrémité proximale (3) et une extrémité distale (4), ledit corps (2) comprenant une première partie (5) partant de l'extrémité proximale (3) et une deuxième partie (6) prolongeant la première partie (5), ladite deuxième partie (6) comportant un aménagement (8) destiné à recevoir un fil, **caractérisé en ce que** sur la paroi périphérique de la première partie (5) est appliquée une éminence hélicoïdale (7) qui s'enlace autour de la paroi périphérique de la première partie (5), ladite éminence hélicoïdale (7) ayant un profil arrondi, et **en ce que** la deuxième partie (6) ne comporte pas d'éminence ni hélicoïdale ni autre.

2. Système d'ancrage osseux selon la revendication 1, **caractérisé en ce que** le profil arrondi a un rayon entre 1 et 1,5 mm.

3. Système d'ancrage osseux selon la revendication 1 ou 2, **caractérisé en ce que** la première partie (5) a une longueur de 6 à 16 mm.

4. Système d'ancrage osseux selon l'une quelconque des revendications précédentes, **caractérisé en ce que** la deuxième partie (6) a une longueur de 2 à 5 mm.

5. Système d'ancrage osseux selon l'une quelconque des revendications précédentes, **caractérisé en ce que** le corps (2) a une forme cylindrique ou une forme de cône tronqué et s'effile depuis l'extrémité proximale (3) vers l'extrémité distale (4).

6. Système d'ancrage osseux selon l'une quelconque des revendications précédentes, **caractérisé en ce que** l'aménagement (8) destiné à recevoir un fil est réalisé sous la forme d'un oeillet.

7. Système d'ancrage osseux selon l'une quelconque des revendications précédentes, **caractérisé en ce que** le corps (2) est réalisé d'un seul tenant.

8. Système d'ancrage osseux selon l'une quelconque des revendications précédentes, **caractérisé en ce que** la première partie (5) et la deuxième partie (6) sont des éléments séparés l'un de l'autre, reliés l'un à l'autre, qui sont mobiles, en particulier rotatifs, l'un par rapport à l'autre.

9. Système d'ancrage osseux selon l'une quelconque des revendications précédentes, **caractérisé en ce qu'**au niveau de l'extrémité proximale (3) est prévu un creux (11) pour l'application d'un outil.

10. Système d'ancrage osseux selon la revendication 9, **caractérisé en ce que** le creux (11) a une forme, qui permet à un système d'ancrage osseux (1), implanté dans un os, à être dévissé hors de l'os au moyen d'un outil correspondant, inséré dans le creux (11).

11. Système d'ancrage osseux selon la revendication 10, **caractérisé en ce que** le creux (11) a la forme d'un carré creux ou d'un six pans creux.
